# EUROPEAN PATENT APPLICATION

(11) **EP 4 375 362 A1**
(43) Date of publication of application: **29.05.2024**
(21) Application number: 22209906.1
(22) Date of filing: 28.11.2022
(51) Int. Cl.: C12M 1/24, C12M 1/12, C12M 3/04, C12M 3/06

(54) **A ROTATING TISSUE CULTURE INSERT FOR THE POSITION INSIDE A ROTATABLE ROLLER BOTTLE PARTIALLY FILLED WITH A LIQUID FOR THE CULTIVATION OF CELLS**

(71) Applicant: Universität Zürich, 8006 Zürich (CH)
(72) Inventor: FIORETTA, Emanuela S., 8952 Schlieren (CH); POULIS, Nikolaos, 8952 Schlieren (CH); EMMERT, Maximilian Y., 8952 Schlieren (CH); HOERSTRUP, Simon P., 8952 Schlieren (CH)
(74) Representative: Kleine, Hubertus

(57) **Abstract**

Rotating tissue culture insert (20, 40) for the position inside a rotatable roller bottle (100) partially filled with a liquid for the cultivation of cells comprising a scaffold (11) provided for the cultivation of cells , **characterized in that** the rotating tissue culture insert (20) comprises an axle body (3) and at least two protrusions extending in radial direction from the axle body (3), wherein the protrusions (4) define wheels of said rotating tissue culture insert (20) with a runway that is in contact with the surface (25) of a roller bottle (100) when the roller bottle (100) is in a lying position.

## Description

### Technical Field

The current invention relates to a rotating tissue culture insert for the position inside a rotatable roller bottle partially filled with a liquid for the cultivation of cells.

Roller bottles are commonly used for about a decade or even longer as method and in a machine for the cultivation of cells. Usually, roller bottles are used for cell growth. In some rare applications said roller bottles may comprise a liquid for the cultivation of cells with nutrients and/or cells and a scaffold for the deposition of said cells. Said roller bottle is usually used for the cultivation of cells in a lying position. That means that the roller bottle is rotated around a horizontal rotation axis that is parallel to the longitudinal axis of the bottle, which is commonly cylindrical.

After the growth of a tissue at the surface of the scaffold, cells are removed in a subsequent step by decellularization. Said tissue, without living cells can be used in various medical applications, such as for implantation or as a dressing or the like, without immunicompatibility issures. In some applications there is a demand for a modelized tissue for the replacement of organs. In some other applications there is also a demand of tissue with a specific collagen orientation.

The inventive rotating tissue culture insert can be for the fabrication of tissue for all the aforementioned applications.

### Background Art

Roller bottles and an apparatus for the movement of roller bottles are well known in the art. The state-of-the art technology in this field has focused thus far on adaptation of the roller bottle itself, as disclosed in EP 0 394 713 A1. This document discloses a porous polystyrene foam being the scaffold.

Further methods for engineering tissues are disclosed by AU 2013277275 B2 and by AU 2017202721 A1.

A further bioreactor for the production of is disclosed by US 8 192 981 B2 for the manufacturing of (stented) TEHVs.

### Object of the invention

It is one first object of the current invention to provide the possibility to impose a preferential ECM orientation in longitudinal direction.

It is one second object of the current invention to provide the possibility to impose a preferential ECM orientation in circumferential direction.

A third object of the current invention is to enhance medium perfusion and oxygenation.

It is a fourth object of the current invention to simplify a method of using a roller bottle for the cultivation of cell-based tissue constructs in comparison to today's standards in this field of technology. Said tissues comprise all cell-based matrix constructs which can potentially be produced by the method described hereinafter.

### Disclosure of the invention

An inventive rotating tissue culture insert, short RTCI, is adapted for the position inside a rotatable roller bottle which is partially filled with a liquid for the cultivation of cells. Said bottle is not part of the inventive insert. The rotating insert is adaptable to rotate inside said roller bottle. The roller bottle is typically in a lying position, in other words the bottle has a horizontal orientation.

A tissue culture insert is provided with a surface, typically a scaffold, on which the cells can accumulate and/or grow. Said insert comprises a scaffold provided for the cultivation of cells preferably at its surface but also into the scaffold material. Said scaffold can preferably formed from biodegradable material that degrades during the formation and growth of the tissue.

The RTCI may further comprise a scaffold holder for holding said scaffold.

Said scaffold holder can preferably be a mesh-like cylindrical support used to hold the scaffold. The scaffold holder can more preferably be a nitinol stent. The nitinol stent might be covered by a non-adhesive coating and/or layer to favour scaffold removal. The non-adhesive layer can be preferably made of parafilm.

The nitinol stent used as an integral part of the production process may in the end product serve as an anchoring system such as an implantation stent system for clinical use. The nitinol material is merely one preferred example for a stent.

According to the invention the rotating tissue culture insert comprises an axle body and at least two protrusions extending in radial direction from the axle body. Further parts, such as a scaffold holder might also be part of said rotating tissue culture insert.

The protrusions form two wheels of said RTCI. Said wheels are provided with a runway that is in contact with the surface of a roller bottle when the roller bottle is in an essentially lying resp. horizontal position.

The invention provides the ability to create a tissue engineered product with a desired preferential collagen orientation by using a RTCI in combination to a roller bottle system. It further allows the rotation of the scaffold with the roller bottle, to enhance medium perfusion and oxygenation and/or enables to impose a preferential ECM orientation via static stretch.

According to a preferred embodiment the RTCI can be used to provide a preferential longitudinal ECM orientation or in another preferred embodiment the RTCI may apply a static circumferential stretch over tissue culture time to induce a preferential circumferential ECM orientation.

A preferred application is the use of the insert for the manufacturing in the cardiovascular tissue engineering field, such as the production of a tissue engineered heart valve, short TEHV.

Further embodiments are subject-matter of the dependent claims.

For the use of the maximal surface of the insert, it is of advantage that at least two protrusions are at a distal position of said axle body. A distal position is an end position of a body.

The maximal radial height of the protrusions is larger than thickness of the scaffold. In the case that an additional substrate also referred to as scaffold holder for holding the scaffold is used, which is positioned over the outer surface of said axle body, the radial dimensions of said protrusions is larger than the sum of the thickness of the scaffold and the substrate. A further optional part of said insert might be a sleeve such as a multipart sleeve, being positioned coaxially to said axle body. If said sleeve is part of said axle body, then said protrusions are larger than the sum of the scaffold, the substrate and said sleeve.

It is of advantage if the substrate and/or the sleeve comprises a plurality of openings. Meshes are also understood as openings in the context of the current inventions and are preferred as a material for the substrate holder. Said substrate is also referred to as a scaffold holder, preferably a demountable part of said rotating tissue culture insert. Said scaffold holder can be individually designed according to the form, size and function of an implant to be formed by a combination of the tissue and the substrate. In this case, the substrate or in other words the scaffold holder can form an arrangement together with the tissue, which can be demounted from the rotating tissue culture insert after the formation of the tissue.

Alternatively, or additionally the sleeve, being preferably a multipart-sleeve is provided with a longitudinal axis parallel to said axle body and comprises a plurality of openings and wherein the scaffold holder is preferably positioned over the sleeve. If there is no sleeve the scaffold holder can be positioned over the axle body and is more preferably part of the rotating tissue culture insert
Alternatively, the formed tissue can be detached from the substrate and can be used for implantation or other medical use without the scaffold holder.

For optimal rotation properties the axle body is a hollow axle body. For an optimum contact of the tissue with the liquid inside the roller bottle said hollow axle body may be provided with a plurality of openings.

Since the scaffold and/or the substrate may have different thickness depending on the tissue to be formed, the protrusions are demountable from the axle body and preferably connected to the axle body by a thread. This way an axle body can be provided with protrusions of different dimensions. Thus, the rotating tissue culture insert can be designed in different dimension by using the same axle body.

Preferably the protrusions have the form of circumferential rings.

The scaffold may further comprise a nonwoven layer so that the liquid can easily penetrate the surface of the scaffold. Preferably said nonwoven can be made of biodegradable material. It may comprise or consist of polyglycolic acid.

In a further preferred embodiment said nonwoven layer can be provided with a coating, more preferable with a biodegradable coating, most preferable with a polyhydroxybutyrate-film.

Alternatively, or advantageously to the idea of the protrusions forming two wheels, the rotating tissue culture insert can be provided as a stretcher for stretching the scaffold in radial direction.

This way the system allows for the possibility to easily adjust the rotating tissue culture insert size in relation to the approach, i.e.: smaller or larger stent-like scaffold holders or in other words support structures can be used to achieve the desired outcome, without the need to change the roller bottle system. Moreover, disposable roller bottle flasks are easy to use, largely available, and can accommodate a great variety of scaffold sizes.

Preferred embodiments of the previously described invention and/or embodiments in advantageous combination with the idea of the protrusions in form of wheels, are described by the further dependent claims.

It is of advantage that axle body comprises a longitudinal axis and wherein the rotating tissue culture insert further comprises one or more parts that are radially movable in relation to said longitudinal axis.

In an advantageous constructional concept, the rotating tissue culture insert may comprise an adjustment mechanism for the adjustment of the radial distance of said one or more part in relation to said longitudinal axis of said axle body.

For a better distribution of the force applied to the scaffold, said one or more parts are arcuated plates.

For a better contact of the scaffold with the liquid said arcuated plates might be provided with openings.

In a preferred embodiment said axle body can be part of said adjustment mechanism and is formed by at least two elements that are linear movable to each other and wherein said elements preferably comprise conical segments at each said element. Said adjustment mechanism can be adjustable by screwing or in other word by a screwing mechanism.

In a further embodiment of the invention or as an independent inventive idea the substrate can be essentially cylindrical and may comprise different sections of bendability, preferably bendability in radial direction, more preferable different sections of bendability in a distal position of said substrate.

This might be achieved for example by a mesh structure comprising different mesh sizes.

A further idea of the invention is the provision of a method for the fabrication of an implant wherein the method comprises the following steps:
A: Providing a roller bottle comprising a liquid for the cultivation of cells and a rotating tissue culture insert comprising a rotating tissue culture insert provided with a scaffold, wherein the roller bottle is in a lying position, such that the fill level of the roller bottle is essentially parallel to the longitudinal axis of said bottle. Said RTCI may be the inventive RTCI as previously described.

The rotating tissue culture insert further comprises a substrate also referred to as a scaffold holder for the attachment of the scaffold at the rotating tissue culture insert.
B: Rotation of said roller bottle, wherein the scaffold is distanced towards the wall of said bottle and wherein said insert is rotating, preferably counter-clockwise to the direction of rotation of said roller bottle, and wherein a tissue is formed at the surface of the scaffold during the rotation of the roller bottle;
C: Removal of an arrangement comprising the tissue and the substrate from the rotating tissue culture insert;

According to the invention said arrangement is provided for the formation an implant. This means that the arrangement is already the implant or can be transformed to be an implant.

The aforementioned arrangement may comprise an essentially cylindrical section wherein radial diameter of the rotating tissue culture insert is adjustable and wherein said radial diameter of the rotating tissue culture insert is adjusted to a variable diameter of said cylindrical section.

### Brief Description of the drawings

Some advantageous embodiments for an inventive rotating tissue culture insert and an inventive embodiment of a method for fabrication are further explained in detail below together with drawings. Specific parts of the different embodiments can be understood as separate features that can also be realized in other embodiments of the invention. The combination of features described by the embodiment shall not be understood as a limitation for the invention.

### Mode for Carrying out the invention

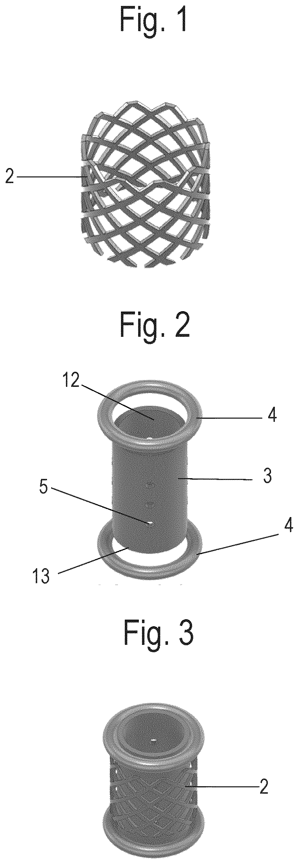

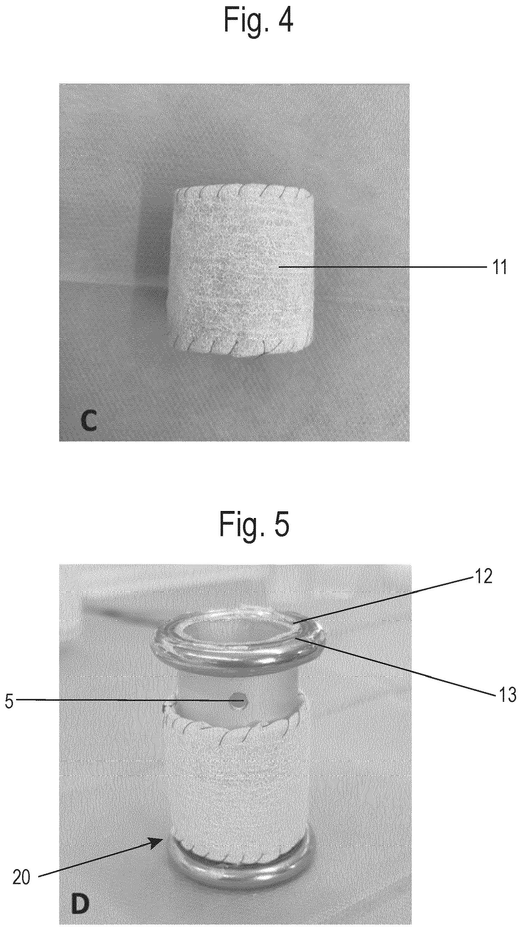

Fig. 1 discloses a first embodiment with a substrate 2 for the support of a scaffold 11 preferably in the form of a stent. Said substrate 2 of this embodiment has the preferred form of a cylindrical mesh body. The substrate 2 can be combined with further parts to a rotating tissue culture insert 20 as shown in Fig. 5.

The scaffold 11 can be mounted to the substrate 2 of the specific form shown in Fig. 1 by using a medical thread by sewing or the like, although other methods of fixation are also possible.

Said scaffold rotating tissue culture insert 20 further comprises a hollow axle body 3 as shown in Fig. 2. Said substrate 2 can be positioned over the outer surface of said hollow axle body 3.

The rotating tissue culture insert is provided with at least two rolling protrusions 4 which are positioned parallel to each other at the hollow axle body. These protrusions 4 are preferably arranged circumferentially around the hollow axle body 3, thus forming a ring shape. Said protrusions 4 are firmly connected to said hollow axle body 3

Each of the protrusions 4 has at least a circumferential runway on which the respective rolling protrusions can be in contact and can roll in contact with a surface, such as the inner wall of a roller bottle. Said runway can be interrupted by grooves. The protrusions 4 are provided to be wheels which are firmly connected to said axle body 3. The substrate 2 and the scaffold 11 can be placed between the protrusions, wherein the radial height of the protrusions is bigger than the sum of the thickness or - in other words - the radial height of the substrate and the scaffold combined.

At least two of said protrusions 4 can be mounted at each end of said hollow axle body 3. They are in a distal position. In a preferred embodiment the mounting said protrusions can be done either by transition or interference fit, by screwing and/or by a combination of clearance fit and welding.

Further protrusions, preferably having an identical form to the said protrusions 4, can be mounted preferably with equidistant from each other and from the distal positioned protrusions 4.

As shown in Fig. 2 said hollow axle body 3 is provided with openings 5 in the shell surface. The openings are preferably arranged in a raw which runs parallel to the longitudinal axis of said hollow axle body. One opening 5 of one raw is equidistant to the neighboring openings of said raw. There are a number of rows of openings circumferentially distributed over the shell surface of said hollow axle body 3.

The material of some parts, preferably all parts of said rotating tissue culture insert 20 might be a metal, preferably of stainless steel for medial use.

The scaffold 11 is shown in Fig. 4. It can be made of a polymeric material, preferably a nonwoven, more preferably comprising polyglycolic acid (PGA). Said PGAnonwoven might preferably be coated with a poly-4-hydroxybutyrate as described later on as a biodegradable film for the control of the cell matrix adhesion. The distal ends 12 is provided with threads 13. The scaffold 11 has the preferred form of a hollow cylinder but might have an alternative prismatic form.

Fig. 5 discloses a rotating tissue culture insert 20, briefly called RTCI, which comprises said scaffold 11. This insert can be used in a rotating cell culture system, RCCS, where a rotating bioreactor is used for the growth of the cells. Said rotating bioreactor can be formed by a roller bottle, as shown for example in EP 0 394 713. The invention is not limited to the use of the RTCI in the roller bottles disclosed in this document.

Roller bottles are known in the art. These containers re used in the laboratory and like situations for culturing of cells. They are generally cylindrically shaped and are adapted to rotate about their axes. The internal surfaces of such roller bottles are for providing active surfaces for the growth of cells. A liquid growth medium is introduced into the roller bottles. The rotating movement of the bottle keeps the internal surfaces wetted with the liquid medium, thereby encouraging the growth of cells. Rotating rollers in an appropriate apparatus are employed to rotate these roller bottles. Usually, the roller bottle apparatus is adapted to be placed inside an incubator or incubating room to control the temperature of cell growth inside the roller bottles. A rotational driving system, such as Rotating rollers, as a part of said apparatus is employed to rotate these roller bottles.

By turning the roller bottle with said RTCI, the RTCI will rotate counter-clockwise or clockwise to the direction of rotation of the roller bottle inside said roller bottle.

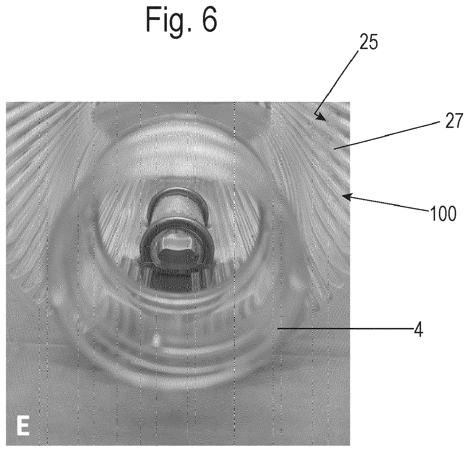

Fig. 6 discloses the inner surface 25 of such a roller bottle 100. The inner surface is provided with ribbons 27 extending parallel to the axis of rotation and the longitudinal axis of said roller bottle 100. Said ribbons 27 are provided for stopping the rotation of the RTCI once the roller bottle is stopped. An axle body with protrusions 4 is here shown made of transparent plastic material to show the arrangement and function of the RTCI inside the roller bottle 100.

Said roller bottle is preferably provided with a neck, such that the bottle is able to withhold of the liquid for cultivating cells in horizontal position. The RTCI is designed in a dimension such that the RTCI preferably rotates at least 2,5 times, preferably 3-4 times around its axis, while the bottle performs one rotation. The RTCI should be in a dimension that it passes said neck of said roller bottle.

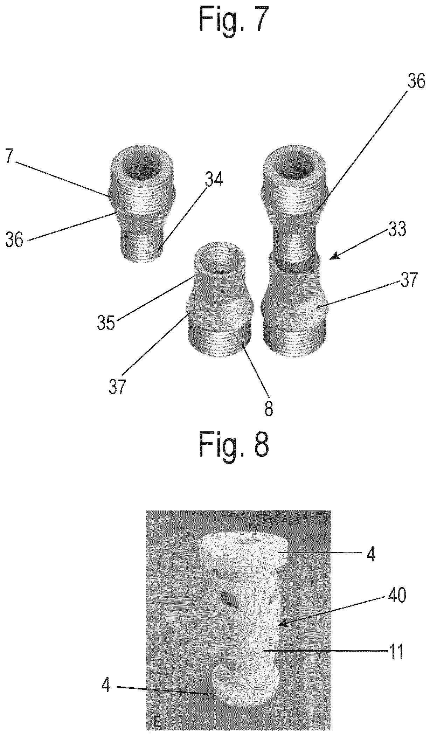

Fig. 7-11 discloses a second embodiment of the current invention, wherein a multipart sleeve 32 is a part of an made of a further embodiment of the rotating tissue culture insert 40 comprising arcuated plates 6 as a part of a jacket section. Each plate is provided with a number of openings 5, preferably at least a row of openings 5. A scaffold holder, or in other words a substrate, can be placed over the sleeve 32.

In the embodiment of Fig. 7-11 there are four arcuated plates 6, however there could be another number of plates thus forming said sleeve. Said sleeve cooperates with an axle body 33, for the adjusting the diameter of said sleeve 32 and the gap between said plates 6. The axle body 33 is formed by an arrangement of at least two parts 7, 8 which are linear movable to each other. In the current invention said linear movability can be achieved by screwing the two parts 7, 8 together. Therefore, one part 7 is provided with a screw bolt 34 and the second part is provided with a screw sleeve 35. The previously described screwing connection of said parts can also be achieved by similar arrangements such as one central screw pin and two distal screw sleeves or by one central screw sleeve and two distal parts provided with screw bolts.

Two parts 7, 8 of said arrangement are provided with conical sections 36, 37 which axial distance can be varied, preferably by screwing or unscrewing said parts. When approaching the conical sections 36, 37 said arcuated plates 6 are spread or respectively are further spaced apart from each other and thus have a higher radial distance from each other compared to the case that the conical sections 36, 37 are further spaced apart from each other.

Because there is sufficient space between axle body 33 and the sleeve 32 formed by the actuated plates 6, said axle body 33 does not need to have a hollow shape or openings 28 of the rotating tissue culture insert 40 of Fig. 7-11 compared to the embodiment of a rotating tissue culture insert 20 of Fig. 1-6, although a hollow shape of the axle body 33 is also preferred in this embodiment.

At the distal positions of the axle body 33 is provided with a thread for mounting rolling protrusions 4 similar to Fig. 1-6. This way the protrusions can be demounted and cleaned independently for reuse. Especially the connection parts are difficult to clean in an acceptable way, therefore a demountability of all parts of the RTCI is preferred. Said parts including the scaffold 11 are shown in Fig. 11 and mounted to an RTCI in Fig. 13.

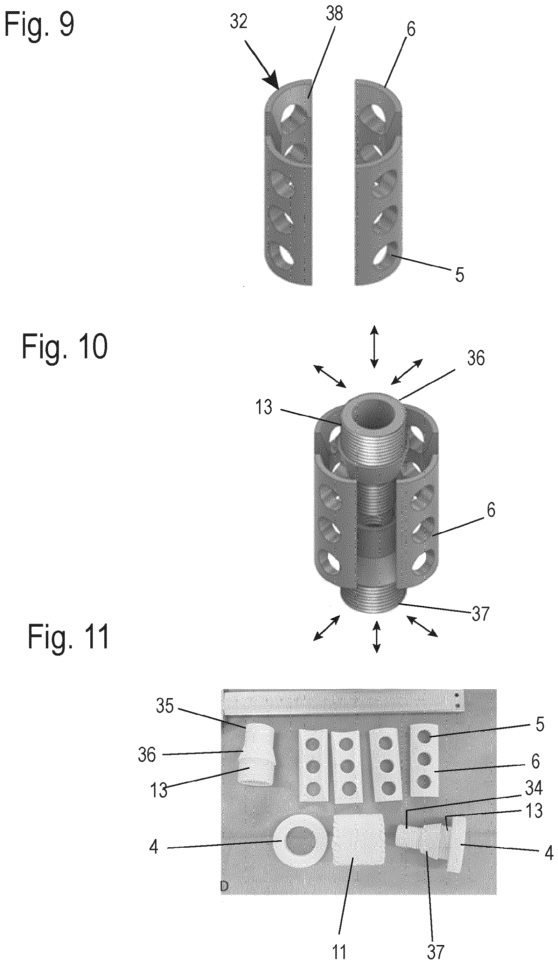

According to Fig. 9 each arcuated plate 6 is provided with a slant 38 at both ends of the plate 6, thus reducing the plate thickness at this position. The slant 38 provides a sliding surface, which is in contact with the conical sections 36, 37.

Fig. 8 discloses the complete RTCI 40. The scaffold holds the plates 6 from falling apart. Wherein the conical parts perform a stretching force on said plates.

The predescribed mechanism of the RTCI for a radial stretching of the scaffold 11 is a further feature of the invention which can realized independent from the provision of the protrusions 4. However, the combination of both features provides a combination of two options for a controlled growth of the tissue on the surface of the scaffold 11 within one insert.

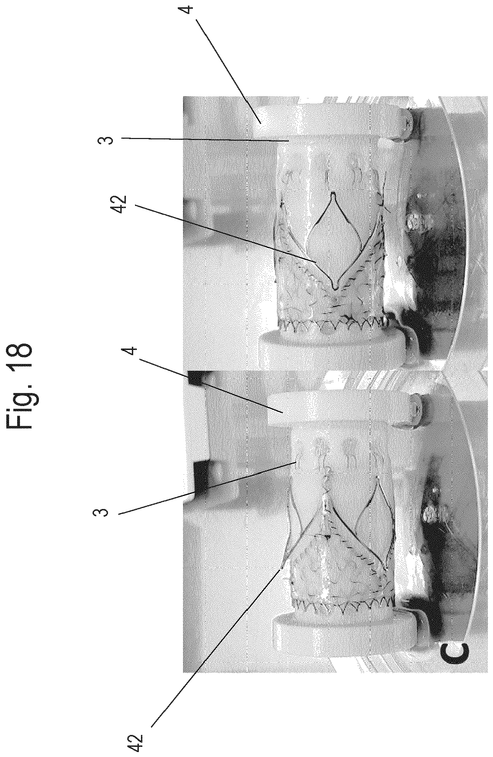

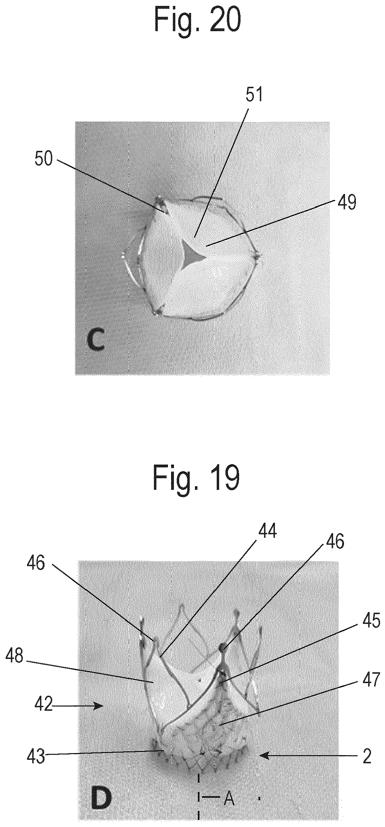

A further object of the invention is the variation of the structure of the substrate 42 in comparison with the aforementioned first and second embodiment as shown in Fig. 18 and 19. The substrate in this case is a cylindrical mesh body as described in the first embodiment, however the substrate 2 is provided with meshes of different sizes. The mesh body 42 is provided with a circumferential mesh section 43 defining a central axis A with meshes having approximately uniform size. The mesh body 42 is further provided with prongs 44, 45 and a having a tapered end 46, wherein the prongs 44, 45 extend parallel to the central axis A from the mesh section 43 towards its end. One prong 44 is has one large mesh 48 having a mesh size of at least 3 times as big as the average mesh size of the meshes of the mesh section 43. The second prong 45 is provided by a mesh structure 47 with meshes having the same average mesh size as the mesh section 43.

This way the second prong 45 is stiffer than the first prong 44. Once the tissue 51 is formed at the surface or around the substrate 42, the section 49 of the tissue 51 formed at or around the surface of the first prongs 44 are more bendable towards the central axis A than the section 50 of the tissue 51 formed at or around the surface of the second prongs 45. As shown valve flaps are realized by modification of the structure 42 as shown in Fig. 20.

Further a use case, further advantages of the invention and some further embodiments are further described below.

Cardiovascular tissues, such as blood vessels and heart valves, are characterized by highly organized extracellular matrix, also called ECM. Among others, the ECM orientation is crucial in determining the mechanical properties of the tissue and its capability in sustaining the hemodynamic loading. Usually, to achieve a strong tissue engineered (TE) construct compatible with cardiovascular tissue engineering applications, such as TE heart valves, TEHV or vascular grafts, complex bioreactor systems are used to mechanically condition the cells by applying physiological-like flow and/or pressure conditions that lead to preferential collagen orientation.

The invention provides a novel and simpler method to produce TE samples with the ability to control the preferential ECM orientation. To fulfill this aim, a new tissue culture method compatible with commercially available roller bottle systems, such as the commercially available system "CellRoll" of the Pfeiffer company, has been developed to favor homogeneous ECM deposition over tissue culture time by ensuring medium perfusion and oxygenation. This new culture method is performed in combination with three different options designed to control ECM orientation and achieve mechanically robust TEHVs:
A first option is the provision of a RTC insert, also called RTCI, wherein the aforementioned protrusions have the function of two wheels as shown in the first, second and third embodiment. This system allows a preferential longitudinal ECM alignment in response to the static stretch determined by the way the scaffold is sutured or fixed otherwise onto the scaffold holder. The resulting TEM can be used for TEHV manufacturing.

A second option is the provision of a stretcher in radial direction for example with a screw-like mechanism and sliding components to manually increase its diameter and an expandable stent-like scaffold holder where the scaffold is sutured onto or otherwise fixed. By imposing of a continuous or step-wise increase of the stretcher diameter over tissue culture time, a preferential circumferential ECM orientation can be achieved. The resulting TEM can be used for TEHV manufacturing. Said sleeve can be part of the stretcher cooperating with said axle body.

A third option the provision of the stent-like scaffold holder which used is a nitinol stent specifically designed for minimally invasive transcatheter aortic valve replacement, short TAVR, techniques. This option will allow the tissue engineered matrix (TEM) to grow directly on the TAVR stent that will be used for implantation, thereby significantly simplifying TEHV manufacturing by reducing TEM handling and suturing or other kind of fixation.

The resulting RTCI is then seeded with cells and inserted in a preferably pleated roller-bottle for tissue culture in a commercially available roller bottle system.

As mentioned before some or all parts of the rotating tissue culture insert of the aforementioned embodiments could be made of medical grade materials such as stainless steel. Alternatively, a suitable material can be a 3D-printed polymer material and/or a glass-fiber enriched polymer materials, such as a nylon blend.

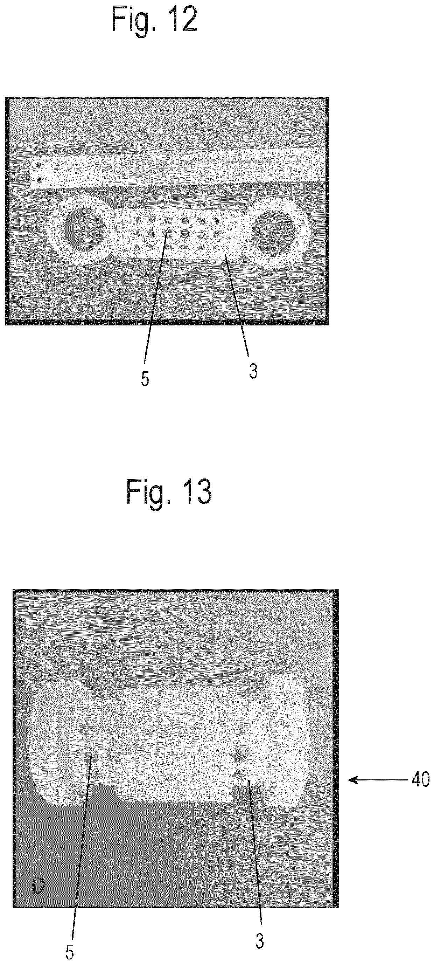

Fig. 12 and 13 show a fourth embodiment of the invention, wherein the hollow axle body 3 is modified such way that it is provided with more openings than in Fig. 1-6. The free space defined by the openings in this embodiment is at least 30% of the outer cylindrical surface of the axle body 3, whereas the free space defined by the openings 5 of the first embodiment is less than 10% of the outer cylindrical surface of the axle body.

Two prototypes of the RTCI for option 1 according to Fig. 1-6 and Fig. 12-13 and one prototype for the RTCI stretcher according to Fig. 7-11 have been manufactured and tested in multiple cultures.

For all the experiments, a scaffold based on polyglycolic acid (PGA) coated with 1% P4HB has been used. For the testing, a rectangular scaffold (size: 9.5 × 4 cm²) was sutured onto a 28 mm stent-like scaffold holder and then inserted either into the RTCI described for option 1 (n=5 for each time point of 2, 4, and 6 weeks of tissue culture) - see Figure 1-6, or into the stretcher described in option 2 (n=4 constructs cultured for 6 weeks) - see Fig. 7-11. Neonatal human dermal fibroblasts (hDFBs, passage 7-9) have been seeded at 1×10⁶ / cm² using fibrin as a cell carrier, as per standard protocol.

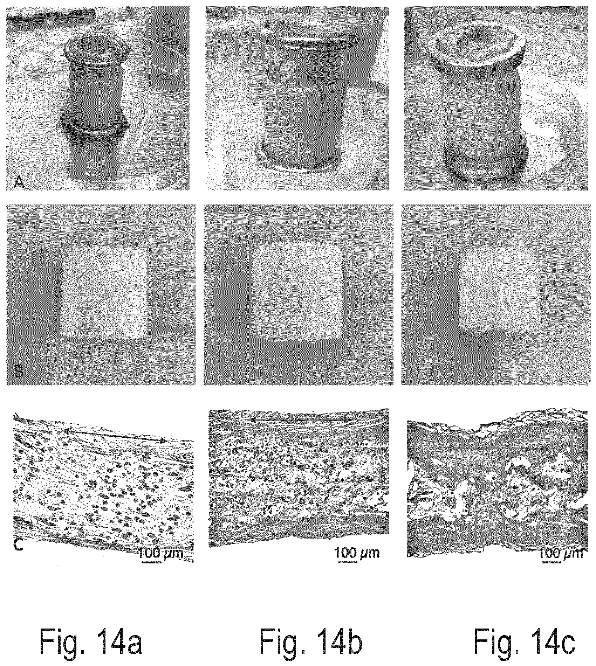

The Results using option 1 are shown in Fig. 14a-14c. TEMs were manufactured using different tissue culture time points to better evaluate the performance of the rotating tissue culture method using the RTCI described in option 1. The following samples have been manufactured and tested by gross evaluation, immunohistology, mass spectroscopy, and uniaxial mechanical testing.
Fig. 14a discloses n=6 tubes cultured for 2 weeks
Fig. 14b discloses n=6 tubes cultured for 4 weeks
Fig. 14c discloses n=6 tubes cultured for 6 weeks

Fig. 14 a-c disclose representative histological images of TEM longitudinal cross sections indicating an increase in ECM deposition and organization over tissue culture time. Importantly, ECM is prevalently aligned in the longitudinal direction, as indicated by the blue arrow.

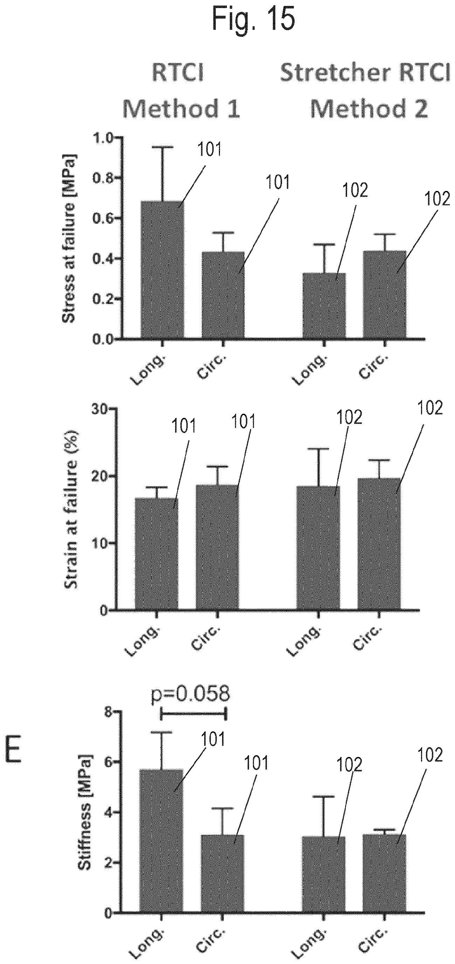

Further the results using option 2 and option 1 were compared. To compare the performance of the RTCI of Fig. 1-6 and the stretcher RTCI of Fig. 7-11, TEMs were manufactured using 6 weeks tissue culture time. N=4 TEM samples have been manufactured using the stretcher RTCI, and tested by gross evaluation, immunohistology, and uniaxial mechanical testing and compared to the results of Fig. 14a-c. Results were compared to the TEMs cultured using the RTCI described in option 1. Fig. 15 shows results after 6 weeks of culture, wherein the resulting TEM is decellularized and removed from the RTCI and scaffold holder for further testing.

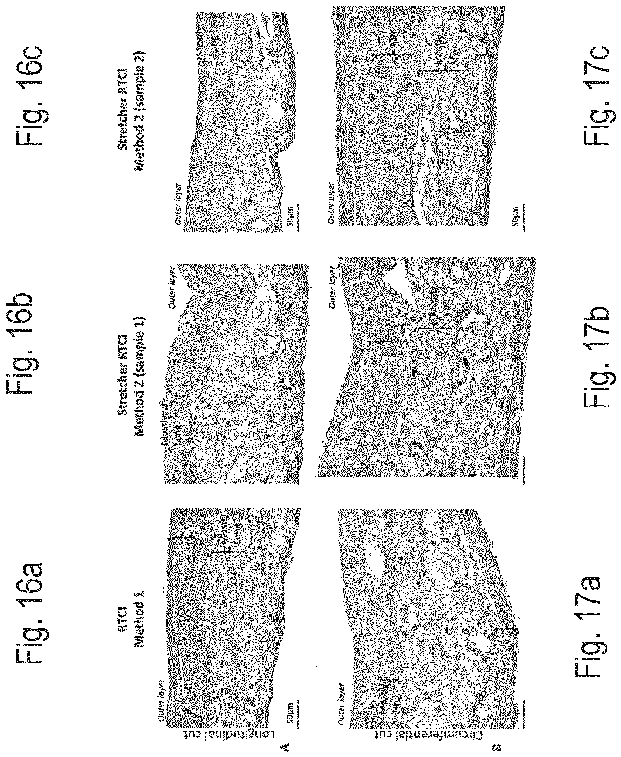

Fig. 15 disclose the Results of the uniaxial tensile test performed on TEM samples manufactured using the stretcher RTCI of Fig. 7-11 having the ref. sign 101 or the RTCI described in option 1 of Fig. 1-6 having the ref. sign 102. Uniaxial mechanical testing of both circumferential and longitudinal samples provided information on tissue anisotropy. Stress at failure and stiffness are greater in the longitudinal direction for TEM tubes cultured using option 1. On the other hand, stress at failure and stiffness are slightly greater in circumferential direction for TEM tubes cultured using the stretcher described in option 2. These results indicate a shift in mechanical properties that can be associated to different ECM orientation as shown in Fig. 16a-c and 17a-c.

Fig. 16 a-c disclose a histological evaluation of samples cultured for 6 weeks using the RTCI of option 1, also called method 1, and the stretcher RTCI of option 2, also called method 2. Said Figures 16 a-c are histological images of samples cut in the longitudinal direction.

Fig. 17 a-c disclose histological images of samples cut in the circumferential direction. This analysis indicates that option 1 results in mostly longitudinal ECM alignment. On the other hand, the stretcher of option 2 or method 2 results in mostly circumferential collagen alignment, in particular within the central portion of the TEM.

Option 3 together with Fig. 18-20 discloses the potential and relevance of the current invention for cardiovascular tissue engineering. Fig. 18-20 is a result after 6 weeks of culture using the a RTCI according to option 1, the resulting TEM was used to manufacture tissue engineered heart valves with TEHVs, n=6, mounted on a nitinol stent compatible with transcatheter aortic valve replacement TAVR-techniques. The valve was then inserted into a custom-made silicon holder with inner diameter of 25mm, and then tested in-vitro in a pulse duplicator system at both pulmonary- (peak systolic pressure: 20mmHg) and aortic-like pressure conditions (peak systolic pressure: 120mmHg) for a minimum of 1 hour with good results. Similarly, after 6 weeks of culture using the RTCI of option 2 the resulting TEM could also be used to manufacture TEHVs, n=2. In vitro testing in the pulse duplicator system indicated good performance at pulmonary pressure conditions for 1 hour.

Next, a detailed way for the preparation of a tissue by using the RTCI is described below:
- Scaffold preparation
   hTEMs were produced as previously described by using non-woven polyglycolic acid (PGA) meshes (thickness 1 mm; specific gravity 70 mg/cm3; Confluent) coated with 1% poly-4-hydroxybutyrate (P4HB, MW 1×10⁶; TEPHA Inc.) in tetrahydrofuran (Sigma-Aldrich) as starting matrix. The PGA/P4HB mesh was cut into scaffolds (width: 100 mm; hight: 45 mm) and then sutured on a cylindrical scaffold holder (30mm in height, 28 mm inner diameter) based on a nitinol stent. Afterwards, the scaffold is combined to the RTC insert according to Fig. 1-6 and the full constructs is sterilized (30 min incubation in 70% ethanol followed by 30 min incubation in PBS supplemented with 10% penicillin-streptomycin (Sigma) and 1% antibiotic-antimicotic solution (Sigma). Finally, the scaffolds were incubated overnight in cell culture medium (Advanced DMEM (Gibco), supplemented with 10% fetal bovine serum (FBS, Gibco), 1% GlutaMax (Gibco), and 1% penicillin-streptomycin (Sigma).
- Cell expansion and seeding
   Human dermal fibroblasts (hDFBs, CellSystems Biotechnologie GmbH, passages 7-10) were plated in roller bottles (850 cm², TufRol EZ, Nunc) and expanded in cell culture medium till confluency using the CELLROLL system. Upon harvesting, cells were seeded onto the scaffolds (1 × 10⁶ cells/cm2) using fibrin as a cell carrier, as previously established. After seeding, the constructs were incubated statically in cell culture medium overnight to favour cell adhesion.
- hTEM culture
   The day after the seeding, the constructs were transferred to a pleated roller-bottle (1450 cm², TufRol, Nunc). Constructs were cultured using 150 ml of tissue culture medium (cell culture medium supplemented with I-ascorbic acid 2-phosphate (0.26 mg/ml; Sigma-Aldrich) from day 1, and 5 ng/ml TGF-β1 (Peprotech), starting at day 7), that was replaced twice a week. Tissue culture occurred using a rotation speed of 1.5 rpm in standard incubator settings (37°C, 5% CO₂, and 95% relative humidity). After 2, 4, or 6 weeks of tissue culture, hTEM tubes were washed in PBS and decellularized using an optimized protocol.
- Decellularization
   hTEM were incubated twice overnight in a detergent solution (0.25% Triton X-100, sodium deoxycholate and 0.02% ethylenediaminetetraacetic acid in DPBS) in the roller bottle. Additionally, four Benzonase (Novagen) incubation steps with decreasing concentration (100, 80, 40 and 20 U/ml in 50 mM TRIS-HCI buffer solution with pH 8.0) were used to degrade the remaining DNA remnants. After rinsing, hTEM tubes were stored in PBS supplemented with 1% penicillin / streptomycin (Sigma) at 4°C until further use.
- (Immuno-)histochemistry
   Qualitative, quantitative, and (semi)quantitative immune-histological evaluation of the cross-sections of the different human-cell derived TEMs (hTEMs) manufactured was used to gain relevant information on ECM structure and deposition, as well as on the presence of scaffold remnants.
   For each tube, a longitudinal portion of the hTEM was cut and fixed in 4% formalin, dehydrated, embedded in paraffin, and cut longitudinally (slice thickness of 3µm). The resulting cross-section was stained using: hematoxylin and eosin (H&E), to evaluate the presence of cells and the tissue morphology; Elastica van Gieson (ELVG) to examine the elastic and collagen fibers; and collagen 1 (COL1, Abcam, ab34710) and collagen 3 (COL3, Abcam, ab7778) to assess the deposition of specific collagens. The stained samples were imaged with the brightfield microscopy (Mirax Midi Microscope, Carl Zeiss GmbH).
- Assessment of hTEM dimensions
   Total hTEM thickness and ECM thickness on the outer and inner layer of the scaffold were measured using the Pannoramic Viewer software (3DHistech, Ltd.). For each hTEM sample, thickness measures were obtained from ten different locations per section and then averaged. For each tissue culture time point (i.e.: 2, 4, and 6 weeks), n=4 different hTEM samples have been measured and the averaged value reported.
- Semi-quantitative evaluation
   A semi-quantitative evaluation of the histology was performed on each sample by grading from 0 (absent) to 5 (abundant) the maturation and presence of ECM (in the outer, inner, and middle region of the hTEM sample), the presence of polymeric remnants, the efficiency of the decellularization, and the presence of collagen 1, 3, and elastin. The analysis was performed by two independent observers for each hTEM sample. For each tissue culture time point (i.e.: 2, 4, and 6 weeks), n=4 different hTEM samples have been analysed.
- Biochemical analysis
   Biochemical assays were used to quantify collagen (via hydroxyproline, HYP) and glycosaminoglycans (GAGs) content. Briefly, 5-8 mg of freeze-dried hTEM samples (n=4 for each time point, in technical triplicate) were digested with papain (Worthington) in a digestion buffer solution and a stepwise protocol was performed to quantify GAGs and HYP content using a plate reader (Tecan infinite M1000 pro) to measure the light absorbance. Quantification was performed by using standards for GAGs and HYP. Double strand DNA (dsDNA) analysis was performed using the Qubit dsDNA HS Assay kit (Thermofisher). The data was normalized to total weight of dry tissue.
- Uniaxial tensile testing
   The uniaxial mechanical testing was performed to gain information on how mechanical properties changes over tissue culture time. hTEM samples in longitudinal direction (n=3 for each tissue culture time point, in technical triplicate) were cut at a length of 9 × 5 mm, clamped into a custom-made clamp before being tested using a uniaxial material testing machine that recorded force-strain data (Zwick Z010 TN, ZwickRoell GmbH, Ulm, Germany - 20N load-cell). During testing, samples were kept in a chamber filled with room temperature PBS. After an initial preload to 0.025 N (0% strain), samples were pre-conditioned five times to 5% strain, with a recovery period of 30s in between each preloading cycle. Then, samples were ramped to failure at a constant strain rate of 100% strain per min. Stress calculation was achieved by using an estimate of the sample cross-sectional area calculated as follow: sample width (5 mm) multiplied by tissue thickness. Tissue thickness was obtained on the basis of histological images of adjacent samples. The thickness value was then corrected by a volume shrinkage correction factor of 1.15 to compensate for loss of tissue volume due histological sample preparation. Sample stiffness (E, Young's modulus), stress at failure and strain at failure were calculated in the linear region of the force-strain curves.
- Heart valve manufacturing and in vitro testing
   Systematic in-vitro assessment of TEHV performance (n=4) was carried out by using a pulse duplicator system HDT-500 (BDC Labs, USA) equipped with a transonic sensor TS410 (Transonic Systems, USA) for flow measurements and a BDC-PT pressure sensor (BDC Labs, USA). All TEHVs were firstly tested at simulated pulmonary pressure conditions (peak pressure: 20 mmHg, mean pressure: 10 mmHg, minimum pressure: 0 mmHg) for 1 hour. After a gradual increase of the applied pressure, the TEHVs were tested at simulated aortic pressure conditions (peak pressure: 120mmHg, mean pressure: 100mmHg, minimum pressure: 80mmHg) for 1 hour. Testing was performed in PBS supplemented with 0.05% Xanthan Gum (Sigma), in accordance with ISO5840 testing requirement. Data were collected for 3 seconds and functionality was assessed by averaging 20 simulated cardiac cycles using the Statys software (BDC Labs, USA) to determine the closing volume (CV, back flow during valve closure), leakage volume (LV, ) and regurgitation fraction (RF) expressed as % of the Forward Flow Volume (FFV); the Effective Orifice Area (EOA, estimated using the Gorlin equation, as described in the ISO5840); and maximum and mean transvalvular pulmonary/aortic gradient (*peak-pressure difference,* PPDₘₐₓ and PPDₘₑₐₙ, respectively, calculated as the difference between ventricular pressure and pulmonary / aortic pressure at systole).

### List of references

- 2: scaffold holder
- 3: axle body
- 4: protrusions
- 5: openings
- 6: plates
- 7: axle body part
- 8: axle body part
- 11: scaffold
- 12: distal ends
- 13: threads
- 20: rotating tissue culture insert
- 25: surface
- 27: ribbons
- 32: sleeve
- 33: axle body
- 34: screw bolt
- 35: screw sleeve
- 36: conical section
- 37: conical section
- 38: slant

- 40: RTCI
- 42: mesh body
- 43: mesh section
- 44: prong
- 45: prong
- 46: tapered end
- 47: mesh structure
- 48: large mesh
- 49: section
- 50: section
- 51: tissue
- 100: bottle
- 101: sign
- 102: sign

## Claims

1. Rotating tissue culture insert (20, 40) for the position inside a rotatable roller bottle (100) partially filled with a liquid for the cultivation of cells comprising a scaffold (11) provided for the cultivation of cells **characterized in that** the rotating tissue culture insert (20, 40) further comprises an axle body (3, 33) and at least two protrusions extending in radial direction from the axle body (3, 33), wherein the protrusions (4) define wheels of said rotating tissue culture insert (20, 40) with a runway that is in contact with the surface (25) of a roller bottle (100) when the roller bottle (100) is in a lying position.

2. Rotating tissue culture insert (20, 40) according to claim 1, **characterized in that** the at least two protrusions are at a distal position of said axle body (3, 33).

3. Rotating tissue culture insert (20, 40) according to claim 1 or 2, **characterized in that** the radial height of the protrusions (4) is larger than thickness of the scaffold (11) or of the sum of the thickness of the scaffold (11) and a scaffold holder (2) and an optional sleeve (32) being coaxial to and positioned over the of the axle body (33).

4. Rotating tissue culture insert (20, 40) according to one of the preceding claims, **characterized in that** the sleeve (32) is provided with a longitudinal axis parallel to said axle body (3, 33) comprising a plurality of openings (5) and wherein the scaffold holder (2) is preferably positioned over the sleeve (32) and/or the axle body (3, 33) and is the part of the rotating tissue culture insert (20, 40) and/or **that** the axle body (3, 33) and/or said sleeve (32) is preferably a hollow body, preferably comprising a plurality of openings (5).

5. Rotating tissue culture insert (20, 40) according to one of the preceding claims, **characterized in that** the protrusions (4) are demountable from the axle body (3) and preferably connected to the axle body (3, 33) by a thread (13).

6. Rotating tissue culture insert (20, 40) according to one of the preceding claims, **characterized in that** the protrusions (4) have the form of circumferential rings.

7. Rotating tissue culture insert (20, 40) according to one of the preceding claims, **characterized in that** the scaffold (11) comprises a nonwoven layer, preferably made of biodegradable material and wherein the nonwoven is preferably provided with a coating, more preferable with a biodegradable coating, most preferable with a polyhydroxybutyrate material.

8. Rotating tissue culture insert (20, 40) according to one of the preceding claims, **characterized in that** the rotating tissue culture insert (20, 40) comprises a stretcher for stretching the scaffold (11) in radial direction, wherein said sleeve (32) is preferably part of the stretcher.

9. Rotating tissue culture insert (20, 40) according to one of the preceding claims, **characterized in that** the axle body (3, 33) comprises a longitudinal axis and wherein the rotating tissue culture insert (20, 40) further comprises one or more parts (7, 8) that are radially movable in relation to said longitudinal axis, thus forming said sleeve (32).

10. Rotating tissue culture insert (20, 40) according claim 9, **characterized in that** the rotating tissue culture insert (20, 40) comprises an adjustment mechanism for the adjustment of the radial distance of said one or more part (7, 8) in relation to said longitudinal axis of said axle body (3, 33) and wherein said one or more parts (7, 8) are preferably arcuated plates (6), more preferably arcuated plates (6) provided with openings (5).

11. Rotating tissue culture insert (20, 40) according to one of the preceding claims, **characterized in that** said axle body (3, 33) is part of said adjustment mechanism and is formed by at least two elements that are linear movable to each other and wherein said elements preferably comprise conical segments at each said element.

12. Rotating tissue culture insert (20, 40) according to one of the preceding claims, **characterized in that** the adjustment mechanism is adjustable by screwing.

13. Rotating tissue culture insert (20, 40) according to one of the preceding claims, **characterized in that** the scaffold holder (2) is essentially cylindrical and comprises different sections of bendability, preferably bendability in radial direction, more preferable different sections of bendability in a distal position of said scaffold holder (2).

14. Method for the fabrication of an implant comprising wherein the method comprises the following steps:
A: Providing a roller bottle (100) comprising a liquid for the cultivation of cells and a rotating tissue culture insert (20, 40), preferably according to one of the preceding claims, provided with the scaffold (11), preferably made of biodegradable material, wherein the roller bottle (100) is in a lying position, such that the fill level of the roller bottle (100) is essentially parallel to the longitudinal axis of said bottle (100);
wherein the rotating tissue culture insert (20, 40) further comprises a scaffold holder (2) for the attachment of the scaffold (11) at the rotating tissue culture insert (20, 40);
B: Rotation of said roller bottle (100), wherein the scaffold (11) is distanced towards the wall of said bottle (100) and wherein said rotating tissue culture insert (20, 40) is rotating in said roller bottle (100) wherein a tissue (51) is formed at the surface of the scaffold (11) during the rotation of the roller bottle (100);
C: Removal of an arrangement comprising at least the tissue (51) and the scaffold holder (2) from the axle body (3, 33) of the rotating tissue culture insert (20, 40),
**characterized in that** the arrangement is provided for the formation of being an integral part of the implant.

15. Method according to claim 14, **characterized in that** the arrangement comprises an essentially cylindrical section wherein radial diameter of the rotating tissue culture insert (20, 40) is adjustable and wherein said radial diameter of the rotating tissue culture insert (20, 40) is adjusted to a variable diameter of said cylindrical section.
